# EUROPÄISCHE PATENTSCHRIFT

(11) **EP 0 802 783 B1**
(45) Veröffentlichungstag und Bekanntmachung des Hinweises auf die Patenterteilung: **25.06.2003**
(21) Anmeldenummer: 95902781.4
(22) Anmeldetag: 01.12.1994
(51) Int. Cl.: A61K 7/48, A61K 31/505

(54) **TOPISCHE ZUBEREITUNGEN MIT EINEM GEHALT AN L-ARGININ**
TOPICAL PREPARATIONS CONTAINING L-ARGININE
PREPARATIONS A TENEUR EN L-ARGININE A USAGE LOCAL

(30) Priorität: 02.12.1993 DE 4341001
(43) Veröffentlichungstag der Anmeldung: 29.10.1997
(73) Patentinhaber: Beiersdorf Aktiengesellschaft, 20245 Hamburg (DE)
(72) Erfinder: RIPPKE, Frank, D-20255 Hamburg (DE); SAUERMANN, Gerhard, D-24649 Wiemersdorf (DE); SCHREINER, Volker, D-20259 Hamburg (DE)
(86) Internationale Anmeldenummer: EP9403999
(87) Internationale Veröffentlichungsnummer: WO95015148

(56) Entgegenhaltungen:
- WO-A-91/19478
- DE-A- 2 242 553
- S.T.N., Datenbank Lifferant,KARLSRUHE, DE Datenbank Embase, AN= 75062478, siehe die zusammensetzung
- DATABASE WPI Week 8613, Derwent Publications Ltd., London, GB; AN 86-084406 & JP,A,61 030 509 (SHISEIDO) 12. Februar 1986

## Beschreibung

Die vorliegende Erfindung betrifft kosmetische und dermatologische topische Zubereitungen mit einem Gehalt an L-Arginin und Harnstoff und Folsäure.

Zubereitungen für trockene und schuppende Haut enthalten normalerweise feuchtigkeitsbindende Substanzen wie Glyzerin, Pyrollidoncarbonsäuresalze oder Milchsäure bzw. die Feuchtigkeitsbindung der Hornschicht erhöhende Komponenten wie Harnstoff oder geeignete, die epidermalen Lipidstrukturen in ihrer Funktion unterstützende Lipide wie Cholesterin. Ihre Wirkung ist aber nicht immer befriedigend, insbesondere bei Atopie (Neurodermitis) und Psoriasis.

Eine Kombination von Vitaminen, z.B. Folsäure, mit Aminosäuren, z.B. Arginin, ist bereits bekannt (S.T.N., Datenbank Lieferant,Karlsruhe, DE. Datenbank Embase AN 75062478). Sie dient zur Behandlung von Psoriasis. Weiterhin ist eine Arginin und Harnstoff enthaltende Zubereitung bekannt (Database WPI, Week 8613, Derwent Publications Ltd., London, GB; AN 86-084406 & JP-A-61 030 509 (Shiseido); 12. Februar 1986).

Aufgabe der vorliegenden Erfindung ist somit, der Haut bessere Wirkstoffe zur Verfügung zu stellen, welche trockene, schuppige Zustände der Haut und Altershaut vermeiden helfen oder erträglicher gestalten und beseitigen.

Aufgabe der Erfindung war es insbesondere, Wirkstoffe und topische Zubereitungen mit solchen Wirkstoffen zur Verfügung zu stellen, welche trockene Haut verhindern oder nach dem Auftreten lindern oder schnell zum Abklingen bringen, also zur Prophylaxe und/oder Behandlung geeignet sind.

Trockene Haut und schuppige Haut und Altershaut können als kosmetisch zu behandelnde Störungen angesehen werden. Starkes Schuppen dagegen, insbesondere bei Atopie oder Psoriasis auftretendes Schuppen der trockenen Haut kann auch als schwerwiegendere dermatologische Störung bezeichnet werden.

Diese Aufgabe wird gelöst durch kosmetische und dermatologische topische Zubereitungen mit einem Gehalt an
a) L-Arginin oder dessen Salzen, Säureadditionssalzen, Estern oder Amiden,
b) Harnstoff,
c) Folsäure oder deren Salzen und
d) Antioxidantien.

Gegenstand der Erfindung ist auch die Wirkstoffkombination, bestehend aus a) , b), c) und d):
a) L-Arginin oder dessen Salzen, Säureadditionssalzen, Estern oder Amiden,
b) Harnstoff,
c) Folsäure oder deren Salzen und
d) Antioxidantien.

Sie dient, wie auch die topischen Zubereitungen, zur Verwendung in der Prophylaxe und/oder Behandlung von trockener und schuppiger Haut und der Altershaut, insbesondere der durch endogene oder chronologische Alterung geschädigten Haut, in der Kosmetik und bei Hautkrankheiten, bei denen solche Symptome auftreten, z.B. bei Atopie (Neurodermitis) oder Psoriasis. Gegenstand der Erfindung ist daher auch die erfindungsgemäße Wirkstoffe und topischen Zubereitungen zur Verwendung in der Prophylaxe und/oder Behandlung von trockener und schuppiger Haut und der Altershaut, insbesondere der durch endogene oder chronologische Alterung geschädigten Haut,
in der Kosmetik und bei Hautkrankheiten, bei denen solche Symptome auftreten, z.B. bei Atopie oder Psoriasis.

Die erfindungsgemäßen Kombinationen eignen sich hervorragend zur Behandlung der bei atopischer Dermatitis und Psoriasis sichtbaren und fühlbaren Hautschäden und zur Behandlung bei mit Juckreiz verbundenen Hauterkrankungen.

Damit werden die gestellten Aufgaben gelöst.

Es war für den Fachmann nicht vorauszusehen, daß die erfindungsgemäße Wirkstoffkombination und daß kosmetische oder dermatologische Zubereitungen mit wirksamen Gehalten an den Wirkstoffen gemäß der Erfindung sich hervorragend zur Prophylaxe und Behandlung der angegebenen Hauterscheinungen eignen.

Bevorzugte Salze von L-Arginin sind wasserlösliche Salze, z.B. Natrium-, Kalium- und Ammoniumsalze. Dies gilt auch für die Säureadditionssalze. Geeignete Säureadditionssalze werden mit anorganischen und organischen Säuren erhalten. Bevorzugt werden die Hydrochloride, Sulfate, Acetate, Caprylate oder Zitrate.

Geeignete Ester des L-Arginins sind z.B. solche, die mit kurzkettigen und mittelkettigen Alkoholen erhalten werden, vorzugsweise mono-Alkohole, insbesondere aber Methanol, Ethanol oder Propanol. Bevorzugt werden die Ethylester.

Bevorzugte Amide sind kurz- und mittelkettige mono- und di-Alkylamide.

Alkyle der vorstehenden Substituenten enthalten z.B. bis zu 12, vorzugsweise bis zu 6 Kohlenstoffatome.

L-Arginin und seine Derivate zeichnen sich auch durch ein besonders gutes Hautpenetrationsvermögen aus.

L-Arginin und seine Derivate sind vorzugsweise in Mengen von 0,01 bis 30 Gew.-%, besonders bevorzugt 0,01 bis 10 Gew.-%, insbesondere 0,1 - 7,5 Gew.-%, jeweils bezogen auf die gesamte Zubereitung, in den erfindungsgemäßen kosmetischen und dermatologischen Zubereitungen enthalten.

Der Harnstoffgehalt beträgt beispielsweise 0,01 - 30 Gew.-%, insbesondere 0,1 - 10 Gew.-%, jeweils bezogen auf das Gesamtgewicht der Zubereitungen.

Besonders geeignete Salze der Folsäure sind wasserlösliche Salze, insbesondere Natrium-, Kaliumund Ammoniumsalze.

Folsäure und ihre Salze sind in den erfindungsgemäßen Zubereitungen bevorzugt in Mengen von 0,0001 bis 5 Gew.-%, insbesondere 0,01 bis 1,5 Gew.-%, jeweils bezogen auf das Gesamtgewicht der Zubereitungen, enthalten.

Die erfindungsgemäßen kosmetischen oder dermatologischen topischen Zubereitungen können auf an sich üblichen Formulierungsgrundlagen beruhen und zur Behandlung der Haut im Sinne einer dermatologischen Behandlung oder einer Behandlung im Sinne der pflegenden Kosmetik dienen.

Besonders vorteilhafte Zubereitungen werden ferner erhalten, wenn die erfindungsgemäßen Wirkstoffe mit Antioxidantien kombiniert werden.

Die erfindungsgemäßen Antioxidantien können vorteilhaft aus der Gruppe der üblichen kosmetischen und dermatologischen Antioxidantien gewählt werden, insbesondere aus der Gruppe bestehend aus Tocopherolen und deren Derivaten, besonders α-Tocopherol bzw. α-Tocopherylestern, insbesondere α-Tocopherylacetat, ferner Sesamol, Gallensäurederivaten wie Methyl-, Ethyl-, Propyl-, Amyl-, Butyl- und Laurylgallat, dem Konyferylbenzoat des Benzoeharzes, Nordihydroguajakharzsäure, Nordihydroguajaretsäure, Butylhydroxyanisol, Butylhydroxytoluol, Ascorbinsäure, Citronensäure, Phosphorsäure, Lecithin, Trihydroxybutyrophenon, Carotinen, Vitamin-A und dessen Derivaten, insbesondere Retinylpalmitat, Ascorbinsäure, Ascorbylpalmitat, Dilaurylthiodipropionat, Distearylthiodipropionat, Monoisopropylcitrat, Thiodipropionsäure, EDTA sowie EDTA-Derivaten, Cystein, Glutathion und Ester Harnsäure, Liponsäure und Ester, Carotine, Schwermetallcomplexbildner wie delta-Aminolävulinsäure und Phytinsäure.

Die erfindungsgemäßen kosmetischen oder dermatologischen Zubereitungen enthalten bevorzugt 0,01 bis 10 Gew.-%, insbesondere aber 0,1 bis 6 Gew.-%, bezogen auf das Gesamtgewicht, an einem oder mehreren Stoffen aus der Gruppe der Antioxidantien.

Bevorzugt ist, die erfindungsgemäßen Antioxidantien aus der Gruppe der Tocopherole und deren Derivaten zu wählen.

Insbesondere war nicht vorauszusehen gewesen, daß die erfindungsgemäße Kombination bzw. die genannte Kombination kombiniert mit Antioxidantien die Hornschicht feuchter, weniger schuppig und dehnbar machen, ihre Durchblutung und Versorgung verbessern und die Schutz- und Barrierefunktion gegenüber exogenen Noxen wie UV-Strahlung, Solventien und Tensiden verbessern.

Ferner war nicht vorauszusehen gewesen, daß die Kombinationen zu hautverträglichen Produkten führen bzw. deren Verträglichkeit steigern und bei gesunder Haut nicht in die hauteigene Mikroflora eingreifen würden.

Zur Anwendung werden die Zubereitungen in der für Kosmetika oder Dermatika üblichen Weise auf die Haut in ausreichender Menge aufgebracht.

Besonders bevorzugt sind Haut- und Handpflegepräparationen, Sonnenschutz und After-sun-Präparate, Wasch- und Duschpräparate mit hautpflegender Funktion.

Dermatologische und kosmetische Zubereitungen gemäß der Erfindung können in verschiedener Form vorliegen. So können z.B. wäßrige, alkoholische oder wäßrig-alkoholische Lösungen, Emulsionen vom Typ Öl-in-Wasser (O/W), Emulsionen vom Typ Wasser-in-Öl (W/O), multiple Emulsionen z.B. vom Typ Wasser-in Öl-in-Wasser (W/O/W), Gele, Hydrodispersionen, feste Stifte oder Aerosole die o.g. Wirkstoffkombinationen enthalten.

Die erfindungsgemäßen topischen Zubereitungen können die üblichen Hilfsstoffe wie Emulgatoren und Konservierungsmittel enthalten.

Bevorzugt sind auch solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Äußerst vorteilhaft sind aber auch solche Zubereitungen, welche nach der Lichtexposition auf die Haut aufgetragen werden, also Après-Soleil-Produkte. Es liegt bei solchen Zubereitungen im Ermessen des Fachmannes, ob zusätzliche UV-Filtersubstanzen verwendet werden sollen oder nicht.

Kosmetische Zubereitungen gemäß der Erfindung zum Schütze der Haut vor UV-Strahlen können in verschiedenen Formen vorliegen, wie sie z.B. üblicherweise für diesen Typ von Zubereitungen eingesetzt werden. So können sie z.B. eine wäßrige, alkoholische oder wäßrig alkoholische Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen topischen Zubereitungen können kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Kosmetische und dermatologische Zubereitungen zur Behandlung und Pflege der Haut können als Gele vorliegen, die neben den Wirkstoffen und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Erfindungsgemäße Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen, metastabile Systeme dar und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Feste Stifte gemäß der Erfindung können z.B. natürliche oder synthetische Wachse, Fettalkohole oder Fettsäureester enthalten. Bevorzugt werden Lippenpflegestifte.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure (2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind z.B. zu nennen:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Gegenstand der Erfindung ist auch die Kombination von erfindungsgemäßen Wirkstoffen mit einem oder mehreren UVB-Filtern bzw. erfindungsgemäße kosmetische oder dermatologische Zubereitungen, welche auch einen oder mehrere UVB-Filter enthalten.

Es kann auch von Vorteil sein, die Wirkstoffe mit UVA-Filtern zu kombinieren, die bisher üblicherweise in kosmetischen und/oder dermatologischen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Es werden auch vorteilhafte Zubereitungen erhalten, wenn die erfindungsgenmäßen Wirkstoffe mit UVA- und UVB-Filtern kombiniert werden.

Auch Kombinationen von den erfindungsgemäßen Wirkstoffen mit einem oder mehreren Antioxidantien und einem oder mehreren UVA-Filtern und/oder einem oder mehren UVB-Filtern sind erfindungsgemäß besonders vorteilhaft.

Die kosmetischen oder dermatologischen Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Gegenstand der Erfindung ist auch das Verfahren zur Herstellung der erfindungsgemäßen topischen Zubereitungen, das dadurch gekennzeichnet ist, daß man in an sich bekannter Weise die Wirkstoffe in kosmetische oder dermatologische Formulierungen einarbeitet.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken.

### Beispiel 1

| Sonnengel LF 4 (transparent) | |
|---|---|
| | Gew.-% |
| L-Arginin | 10 |
| Folsäure | 0,1 |
| Harnstoff | 5 |
| Benzophenon-4 | 0,5 |
| Phenylbenzimidazolsulfonsäure | 1,3 |
| Acrylamid/Natriumacrylat-Copolymer | 1,6 |
| Ethanol | 5,0 |
| Glycerin | 15,0 |
| NaOH (15-%ig) | q.s. |
| Parfum, Konservierungsmittel | q.s. |
| Wasser, VES (vollentsalzt) | ad 100,0 |

### Beispiel 2

| Hydrodispersion | |
|---|---|
| | Gew.-% |
| L-Argininhydrochlorid | 5,0 |
| Folsäure | 0,5 |
| Harnstoff | 5 |
| Phenyltrimethicon | 1,0 |
| Carbomer (Carbopol 981) | 1,0 |
| Hydroxypropylmethylcellulose | 0,2 |
| Butylenglycol | 3,0 |
| Tromethamin | q.s. |
| EDTA-Lösung (14-%ig) | 0,5 |
| Ethanol | 5.0 |
| Parfüm, Konservierungsmittel | q.s. |
| Wasser, VES | ad 100,0 |

### Beispiel 3

| Sonnenmilch O/W | |
|---|---|
| | Gew.-% |
| L-Argininhydrochlorid | 2,5 |
| Folsäure | 0,5 |
| Harnstoff | 5,0 |
| Octylmethoxycinnamat | 5,0 |
| Butylmethoxydibenzoylmethan Cetearylalkohol + PEG-40 Rizinusöl | 1,0 |
| + Natriumcetearylsulfat | 2,5 |
| Glyceryllanolat | 1,0 |
| Laurylmethicon Copolyol | 0,5 |
| Mineralöl (DAB 9) | 5,0 |
| Caprylic/capric Triglyceride | 5,0 |
| Acrylamid/natriumacrylat Copolymer | 0,3 |
| Cyclomethicon | 2,0 |
| TiO₂ | 1,0 |
| Glycerin | 3,0 |
| EDTA-Lösung (14-%ig) | 0,5 |
| Ethanol | 5,0 |
| Parfum, Konservierungsmittel | q.s. |
| Wasser, VES | ad 100,0 |

### Beispiel 4

| Pflegelotion W/O | |
|---|---|
| | Gew.-% |
| L-Argininhydrochlorid | 2,5 |
| Folsäure | 0,25 |
| Harnstoff | 5,0 |
| Cyclomethicon | 3,0 |
| PEG-1-Glycerin Sorbitan Oleostearat | 1,7 |
| PEG-7 Hydriertes Rizinusöl | 6,3 |
| Mineralöl (DAB 9) | 13,0 |
| Caprylic/capric Triglyceride | 13,0 |
| Glycerin | 4,0 |
| MgSO₄ | 0,7 |
| Parfum, Konservierungsmittel | q.s. |
| Wasser, VES ad | 100,0 |

### Beispiel 5

| Pflegende Tagescreme O/W | |
|---|---|
| | Gew.-% |
| L-Argininhydrochlorid | 2,5 |
| Folsäure | 0,1 |
| Harnstoff | 5,0 |
| PEG-5 Glycerylstearat | 2,00 |
| Glycerylstearat | 3,00 |
| Cyclomethicon | 3,00 |
| Caprylic/capric Triglyceride | 3,00 |
| Cetylalkohol | 3,00 |
| Ethanol | 1,00 |
| Hyaluronsäure | 0,05 |
| Tocopherylacetat | 0,50 |
| Glycerin | 4,00 |
| Parfum, Konservierungsmittel | q.s. |
| Wasser, VES | ad 100,00 |

### Beispiel 6

| W/O-Creme | |
|---|---|
| | Gew.-% |
| L-Argininhydrochlorid | 2,5 |
| Folsäure | 0,5 |
| Harnstoff | 5,0 |
| PEG-22-Dodecyl Glycol Copolymer | 3,0 |
| Cetyl Dimethicon Copolyol | 2,0 |
| Cyclomethicon | 4,0 |
| Mineralöl (DAB 9) | 4,0 |
| Caprylic/capric Triglyceride | 4,0 |
| Glycerin | 4,00 |
| Parfum, Konservierungsmittel | q.s. |
| Wasser, VES | ad 100,00 |

### Beispiel 7

| After Sun Lotion | |
|---|---|
| | Gew.-% |
| L-Argininhydrochlorid | 5,0 |
| Folsäure | 0,5 |
| Harnstoff | 5,0 |
| Cetearylalkohol + PEG-40 Rizinusöl | |
| + Natriumcetearylsulfat | 2,50 |
| Glycerylstearat SE | 0,60 |
| Mineralöl (DAB 9) | 4,00 |
| Caprylic/capric Triglyceride | 2,00 |
| Schibutter | 2,00 |
| Avocadoöl | 2,00 |
| Tocopherylacetat | 3,00 |
| Acrylamid/natriumacrylat Copolymer | 0,30 |
| Glycerin | 4,00 |
| Hyaluronsäure | 0,05 |
| Bisabolol | 0,05 |
| Parfum, Konservierungsmittel | q.s. |
| Wasser, VES | ad 100,00 |

### Beispiel 8

| Duschmilch | |
|---|---|
| | Gew.-% |
| L-Argininhydrochlorid | 10,0 |
| Harnstoff | 10,0 |
| Folsäure | 1,0 |
| Sodum Laureth Sulfate | 11 |
| Cocamidopropyl Betaine | 5 |
| Cocamide DEA | 1 |
| PEG-8 | 1 |
| Soybean Oil | 1 |
| Citric Acid | 0,1 |
| Sodium Chloride | 0,2 |
| Fragrance | 0,1 |
| Wasser, demin. | ad 100,00 |

## Patentansprüche

1. Kosmetische und dermatologische topische Zubereitungen mit einem Gehalt an
a) L-Arginin oder dessen Salzen, Säureadditionssalzen, Estern oder Amiden,
b) Harnstoff,
c) Folsäure oder deren Salzen und
d) Antioxidantien.

2. Zubereitungen gemäß Anspruch 1, **dadurch gekennzeichnet, daß** die Wirkstoffe a-c in Kombination mit Antioxidantien, ausgewählt aus der Gruppe der Tocopherole, vorliegen.

3. Zubereitungen gemäß Anspruch 1 zur Verwendung in der Prophylaxe und/oder Behandlung von trockener und schuppiger Haut und der Altershaut, insbesondere der durch endogene oder chronologische Alterung geschädigten Haut, in der Kosmetik und bei Hautkrankheiten, insbesondere Atopie oder Psoriasis.

4. Zubereitungen nach Anspruch 1, **gekennzeichnet durch** einen Gehalt von Substanzen, die UV-Strahlung im UVA- und/oder UVB-Bereich absorbieren.

5. Wirkstoffkombination, bestehend aus
a) L-Arginin oder dessen Salzen, Säureadditionssalzen, Estern oder Amiden,
b) Hamstoff,
c) Folsäure oder deren Salzen und
d) Antioxidantien.

6. Wirkstoffkombination gemäß Anspruch 5 zur Verwendung in der Prophylaxe und/oder Behandlung von trockener und schuppiger Haut und der Altershaut, insbesondere der durch endogene oder chronologiache Alterung geschädigten Haut, in der Kosmetik und bei Hautkrankheiten, insbesondere Atopie oder Psoriasis.

## Claims

1. Cosmetic and dermatological topical formulations having a content of
a) L-arginine or salts, acid addition salts, esters or amides thereof,
b) urea,
c) folic acid or salts thereof and
d) antioxidants.

2. Formulations according to Claim 1, **characterized in that** the active compounds a-c are present in combination with antioxidants selected from the group comprising tocopherols..

3. Formulations according to Claim 1 for use in the prophylaxis and/or treatment of dry and scaly skin and of aged skin, in particular skin damaged by endogenous or chronological ageing, in cosmetics and in the case of skin diseases, in particular atopy or psoriasis.

4. Formulations according to Claim 1, **characterized by** a content of substances which absorb UV radiation in the UVA and/or UVB range.

5. Active compound combination comprising
a) L-arginine or salts, acid addition salts, esters or amides thereof,
b) urea,
c) folic acid or salts thereof and
d) antioxidants.

6. Active compound combination according to Claim 5 for use in the prophylaxis and/or treatment of dry and scaly skin and of aged skin, in particular skin damaged by endogenous or chronological ageing, in cosmetics and in the case of skin diseases, in particular atopy or psoriasis.

## Revendications

1. Préparations topiques cosmétiques et dermatologiques contenant
a) de la L-arginine ou ses sels, sels d'addition d'acide, esters ou amides,
b) de l'urée,
c) de l'acide folique ou ses sels, et
d) des antioxydants.

2. Préparations selon la revendication 1, **caractérisées en ce qu'**elles contiennent les agents actifs a à c en combinaison avec des antioxydants choisis parmi le groupe constitué des tocophérols.

3. Préparations selon la revendication 1 destinées à être utilisées dans la prophylaxie et/ou le traitement de la peau sèche et squameuse et du vieillissement de la peau ; en particulier de peaux endommagées par un vieillissements ou chronologique, dans le domaine cosmétique ou en cas de maladies cutanées, en particulier l'atopie ou le psoriasis.

4. Préparations endogène selon la revendication 1, **caractérisées en ce qu'**elles contiennent des substances absorbant le rayonnement UV dans la plage des UVA et/ou des UVB.

5. Combinaison d'agents actifs, constituée :
a) de la L-arginine ou ses sels, sels d'addition d'acide, esters ou amides,
b) d'urée,
c) d'acide folique ou ses sels, et
d) d'antioxydants.

6. Combinaison d'agents actifs selon la revendication 5 destinée à être utilisée pour la prophylaxie et/ou le traitement de la peau sèche et squameuse et du vieillissement de la peau, en particulier de peaux endommagées par un vieillissement endogène ou chronologique, dans le domaine cosmétique ou en cas de maladies cutanées, en particulier l'atopie ou le psoriasis.
